# EUROPEAN PATENT SPECIFICATION

(11) **EP 0 487 480 B1**
(45) Date of publication and mention of the grant of the patent: **21.06.1995**
(21) Application number: 91850287.3
(22) Date of filing: 21.11.1991
(51) Int. Cl.: C07K 2/00

(54) **Method for the manufacture of proteaseinhibitors from potato-fruitwater**
Verfahren zur Herstellung von Proteasehemmstoffen aus Kartoffelfruchtwasser
Procédé de préparation d'inhibiteurs de protéase à partir de jus de pommes de terres

(30) Priority: 22.11.1990 NO 905003
(43) Date of publication of application: 27.05.1992
(73) Proprietor: NORSKE POTETINDUSTRIER AL, N-2801 Gjovik (NO)
(72) Inventor: Borud, Ole Jens, N-2600 Lillehammer (NO)
(74) Representative: Rostovanyi, Peter

(56) References cited:
- WO-A-90/05456
- US-A- 3 461 205

## Description

This invention concerns a method for extracting and concentrating proteaseinhibitors from potato-fruitwater which is a waste from the production of potatostarch.
Proteases are enzymes which hydrolyse proteins into aminoacids, and proteaseinhibitors are compounds which inhibit this reaction.
Proteaseinhibitors preserve raw materials of animal origin and materials which contain or are contaminated by proteases, by inhibiting the hydrolysis of proteins to aminoacids. In this way the proteins are made unavailable to the digestionsystem of the microorganisms.
Owing to a high content of protease, prey-containing fish gives reduction in quality as well as difficulties in processing when used as raw material for the fish-meal and fish-oil industry.
In laboratory experiments and in wholescale tests under actual fishing, it has been found that proteaseinhibitors extracted from potatojuice efficiently hinder the dissolving of proteins and the formation of biogenic amines in prey-containing fish for industrial use. Potato-inhibitors from the juice of the potato make prey-containing fish keep better in storage. For the fishmealindustry this means that problematic rawmaterial of low storageability may be upgraded to production of fishmeal of a better quality than meal produced from fish without this proteaseinhibitor treatment.
Published information is available that the potato contains inhibitors for chymotrypsin, carboxypeptidase A and B and trypsin. By analysing for these proteases we have followed the movement and yield of these inhibitors through different steps of the process which we are going to describe.
The juice of the potato tubers is a waste which gives environmental problems in the production of potato starch. In the professional vocabulary the undiluted juice from the potato tuber is called potato fruitjuice, whereas the diluted juice is designated potato fruitwater. Both have a high content of organic materials which give rise to high oxygen demand (high BOD and COD) in waste water from the potato starch plants. The potato fruit water also contains phosporous- and nitrogen-compounds which fertilize the recipients.

Norwegian potato starch factories and gradually many potato starch factories in other countries use methods whereby the potato fruitwater is extracted with little or practically no dilution.
The procedure for extracting and concentrating proteaseinhibitors, for which procedure protection by patent is applied for, presupposes the use of little diluted fruitjuice as raw material.

Concentrating potato fruitwater to be used as feed supplement by evaporation or reverse osmosis is used by some potato starch manufacturers. Reverse osmosis is not as energy demanding as evaporation, does however demand that the potato fruitwater is pretreated and filtered clear to avoid clogging of the membranes which hold inorganic salts and low molecular organic components back in the concentrate.

Even if the proteaseinhibitor concentrate is primarily intended to be used for the preservation of fish, it may also be used to inhibit unwanted protease activities in other raw materials of animal origin and products with such unwanted activities.

It will also be described a method for further purification and concentration of proteaseinhibitors made from potato fruitjuice.

In the medical litterature it is claimed that some proteaseinhibitors may cure or help cure certain types of illness. The purified and concentrated proteaseinhibitors may be the basis for further fractionation and purification to investigate the possibilities for this type of use.

According to this invention, the raw material for the extraction of proteaseinhibitors is the fruit juice from the production of potato starch. Small scale extraction of proteaseinhibitors from potatoes is described in the litterature. For extraction of potatojuice and production of proteaseinhibitors on a laboratory scale, whole potato tubers have allways been used, and the production of the juice has been done under controlled ideal conditions. It does however turn out that the proteaseinhibitors in question only to a slight degree are destroyed during the industrial starch production when the starch is washed out from the finely ground potatoes with sulfurdioxide containing potatofruitjuice, and that the wastewater after the extraction of the starchgranules is an exellent source for the production of proteaseinhibitors.

The dry matter content of the potatoes varies depending on the potato variety and growthconditons between 20 and 30 g dry matter/100g potato tuber. The dry matter of the potato tubers contains considerable quantities of protein, 5 - 6 g protein/100 g dry matter.
The potatoprotein is composed of a complex group of proteins and polypeptides with strong inhibitor activities towards the most important pancreaseproteases of higher animals.

As a mean, the potato tuber contains 3/4 water and 1/4 dry matter.
The starch and the fibers occur as particulate material, and make up the larger part of the dry matter. The liquid phase has varying content of dissolved dry matter, and the proteaseinhibitors make up part of this dissolved dry matter. The starch is available as granules suspended in the potatojuice which again is enclosed in cells. The extraction of the starch starts by grinding the potato tubers in such a way that the cell walls are broken and the juice with starch granules and fibers is made free and make a slurry. The starch granules and the fibers are afterwards mechanically separated from the potato fruitjuice. The juice contains phenol compounds which on oxydation give coloured compounds. Most noticeable is the oxydation of tyrosin to melanin and further to compounds with brown and black colour.
Addition of sulphite as sulphurdioxidegas (SO₂-gas) or sulphites gives a reducing environment which prevents the formation of coloured compounds, and at the same time sulphite protects the proteaseinhibitors against damage.

In Norwegian potato starch factories the potato tubers are fineground with high speed grating machines, and the starch granules and the fibers separated from the fruitjuice by a method which is developed in Sweden by the inventor Gösta Larsson. The process makes use of rotating conical sieves, nozzle separators and vacuumfilters. The potato fruitjuice is recirculated and used for transporting the slurry and to wash the starch granules through the fine gauze of the sieves.

Sulphurdioxidegas is added to the potatoslurry to prevent oxydation.
Normaly about 0.2 kg sulphurdioxide (SO₂ gas)/tonn potatoes is used.

Undiluted potatofruijuice may also be separated from the finely ground potatomass by the use of decantercentrifuges. Decantercentrifuges are centrifuges with conical, rotating drums where heavy solids move to the periphery and settle to the drum surface and are continuously transported out by means of a screw and leaves the centrifuge. The potatofruitjuice leaves the decantercentrifuge through an adjustable overflow. The solid material is mostly made up of starch and fibers.
The starch may be separated from the fibers and worked up into potato starch, or the mixed solid material from the decantercentrifuge may be used as raw material in potatodistilleries.

The potato fruitjuice separated by both these methods may be used in the production of proteaseinhibitor concentrate by the method described in the patentapplication.

By using the invention, the environmental problems previously stated are reduced for the reason that part of the proteins leaves the plant as product, and because one gets a permeate which is well filtered and easy to concentrate by reverse osmosis.

The purpose of the invention is to make use of the potato fruitjuice, which is a waste, as raw material in the production of proteaseinhibitor concentrate. According to the invention this purpose is obtained by the traits given in the characterizing part of claim 1.
Additional characterizing traits are given in the dependent claim .

The procedures accordning to the invention are in detail described in the following.

### Heat coagulation of inactive proteins

Proteins for human consumption may be obtained from potatofruitjuice.
The proteins are coagulated by making the potatojuice slightly acid and heat it. The coagulated proteins precipitate and the precipitate is afterwards separated using a cecantercentrifuge or in other ways.
The same coagulation method is used as pretreatment before concentrating the potatojuice by evaporation or by the use of membraneprocesses, the purpose being afterwards to mix the concentrate with the coagulate removed from the juice and the fibers separated and drying the mix to produce feeding stuffs. By these processes conditions are used where the aim is to obtain a granular proteincoagulate which may be separated from the clear phase using decantersentrifuges or by filtering. Conditions are used which destroy the proteaseinhibitors. There is no information available indicating the use of heat treatment and removal of the coagulate on a technical scale using conditions which retain the biological activities.

The proteaseinhibitors from potato tubers may be divided into more or less heatstable. It has been found and characterized different proteaseinhibitors among the fairly heatstable. This is divided 3 main groups,
(1) inhibitors with strong chymotrypsininhibitor activity, and
(2) inhibitors which inhibit strongly both chymotrypsin and trypsin, and
(3) a polypeptide which inhibit the activities of carboxypeptidase A and B.
The process which is described here, aim at extracting and concentrating the reasonably heatstable inhibitors from the potatojuice.

The result of the coagulation depends on several variables, most important are the following parameters,
- temperature,
- time of heat treatment,
- the pH of the potatojuice,
- concentration of sulphite in the potatojuice.
When the purpose of the treatment is to precipitate as much protein as possible to be separated and used as food or for consume, hard coagulating conditions are used and a granular coagulate is obtained which is easy to separate from the potato juice. When the aim is to produce a concentrate with good yields of active proteaseinhibitors, it is essential to use mild reaction conditions during the coagulation. Under mild conditions however a coagulate with a soft and slimy structure is obtained which is difficult to separate from the reaction-mixture. We have found that a highspeed plateseparator with nozzle rejection of the concentrate and periodic sludge emptying and cleaning of the sludgechamber at full speed, gives a clear potato juice which does not block the membranes used for the succeeding concentration by ultrafiltration.

The potatofruitjuice contains about 200 mg sulphite (as SO₂)/litre.
Usually sufficient sulphite has been added as sulphurdioxidegas during the starch production. It may however be necessary to adjust the sulphiteconcentration during heat coagulation. To add sodiumsulphite is a practical way to do this.

The pH of the juice is adjusted by controlled addition of hydrochloric acid. To obtain a good mixing the hydrochloric acid is injected in the pipe line transporting the potatofruitjuice to the plant. The addition is automatically controlled by pH measuring and controlled injection of the acid to pH about 4.4.

The heating to coagulation temperature is done continuously in a tube heatexchanger dimensioned for little overtemperatures on the heattransporting surfaces to avoid local overheating which may destroy inhibitor proteins. Using warm water of 75 - 80 °C as heating medium and a coagulation temperature of 65 - 70 °C measured in the warm juice leaving the heat exchanger after heating for 5 - 10 minutes are preferred conditions. A sufficient precipitation of inactive proteins is obtained this way, and at the same time tolerable loss of inhibitoractivity takes place. The conditions depend on potato varieties and the growth conditions used. Formation of coating on the heating surfaces is avoided by processing a not foaming potatojuice, and using a low surfacetemperature of the heatexchanger together with high liquid velocity along the the surface. We have found that a liquid velocity of about 1.6 m/sec gives usable conditions.

A high speed disccentrifuge with nozzle outlet of the heavy concentrate together with periodic rejection of sludge and emptying of the sludgechamber under operation, gives a clear juice which does not block the surface of the membranes used for the following concentration by ultrafilitration.
With small production or with production outside the season with starch production, a season which may often be short, a Westfalia decantercentrifuge with a nominal capacity 3.5 m³/hr has been used for extracting the undiluted fruitjuice from finly ground potatoes. The solid phase containing fibers and starch and some potatojuice has been used as raw material in a nearby distillery. About 60 % of the juice is separated and heated batchwise in a 4 m³ reactiontank provided with stirrer and an outside heating mantel and a heating coil for circulating water of 80 °C as heating medium. The potatojuice was adjusted to pH about 4,4 and 200 mg sulphite (as SO₂)/l added before heating to 65 °C Curing 20 mins. followed by cooling to 20 °C with cold water through the heatechanger mantel and coil. The coagulate was removed with a filterpress using kieselguhr (Hyflo) as filteraid.

### Concentrating by ultrafiltration

After coagulation and removal of inactive proteins, the clear fluid contains the proteaseinhibitors. This clearified potatojuice is concentrated by ultrafiltration using a relatively low pressure-difference through the membranes. To obtain a good capacity without difficulties, it is important to use a high cross-flow, i.e. high liquid velocity along the membransurface, and that the temperature is controlled in such a way that further precipitation of proteins within the membranes does not occur or the membranes are damaged in other ways.

The proteaseinhibitors are held back in the retenate, as water and small molecules moves through the ultrafiltration membranes as permeate. The progress of the concentration step is followed by measurement of the volumes and determination of the inhibitoractvities.
At present the chymotrypsininhibitoractivity is determined. The juice is concentrated to a predetermined activity. The use of 0,25 l proteaseinhibitorconcentrate to 100 hl fish for fishoil and fishmeal production is practical and gives adequate stabilization of the fish rawmaterial.
This corresponds to 10 to 15 times concentration of the juice after pretreatment depending on the quality of the potato rawmaterial.

The concentration is at present done by the use of equipment from DDS-FILTRATION, Denmark, Type 3 x 38 H-5, membranes type ETNA 10 A (polysulfon) and a membranearea of 105 m². The cut-off of the membranes is molweight about 10 000 Dalton. Using 4 bar pressure drop through the membrares in the module (5 bar on the inletside and 1 bar on the permeateside), 30 °C and recirculating about 45 m³/hr over the membranes in the module, the flux on starting up is about 70 l permeate/m²/hr.

### Preserving and storing the concentrate

The proteaseinhibitorconcentrate is made nonperishable by the addition of 8% common salt (NaCl), together with the inorganic salts already present in the potatofruitjuice, a wateractivity sufficient to prevent growth of most microorganisms is obtained. The concentrate is stored at low temperature, about 10 °C, and keeps for two years.

### Further purification and concentration of the proteaseinhibitorconcentrate

It is known that proteins, also proteaseinhibitors, precipitate by the addition of salts. We have found that common salt (NaCl) is suitable for the precipitation of proteaseinhibitors from potatoes.
This is advantageous as the proteaseinhibitorconcentrate already is preserved by the addition of common salt. By adding adding 150 g salt/l to a concentrate preserved with 80 g salt/l at 10 °C, the proteaseinhibitors precipitate from solution. The precipitate i removed by centrifugation, washed with a pure solution of salt and filtered under vacuum. The precipitate is dissolved in water and filtered, and salt removed by dialysis or diafiltration. The solution is then concentrated by evaporation under vacuum at 35 °C to a sirup.
The remaining water is removed from the sirup by freezedrying.
A dry product is obtained where 20 % of the protein is proteaseinhibitorprotein.

## Claims

1. A method for producing proteaseinhibitors CHARACTERIZED THEREBY that as raw material for the process, potatojuice from the production of potato starch is used in such a way that the process includes,
- addition of not less than 200 p p m sulphurdioxide or sulphite to prevent oxydation during the production of potato starch or that sulphurdioxide or sulphite is added to the potatojuice during the production of the proteaseinhibitors,
- adjusting the pH to 4,6 or lower,
- heating to 65 - 70 °C, which is the coagulation temperature of the inactive proteins (globulins) in the potatojuice, with heatexchangers with a controlled surfacetemperature of 75 - 80 °C so that lokal overheating does not damage the proteaseinhibitorproteins,
- cooling the reactionmixture after the heatcoagulation with a coolingheatexchanger,
- using a highspeed nozzleseparator with a system for throwing away the sludge to get rid of the soft and slimy coagulate,
- collection and transport of the clear phase from the nozzleseparator to concentration,
- removal of water by ultrafiltration at about 4 bar pressurefall through the membranes and high cross flow over the membranes untill the predetermined concentration is obtained, the development of the concentration being followed by measurement of volumes and determination of inhibitoractivities, and
- addition of 7 - 8 % common salt (NaCl) for preserving the proteins and as part of subsequent saltaddition necessary to precipitate active proteins should so be desired.

2. The method according to claim 1, CHARACTERIZED thereby that the procedure also includes production of purified proteaseinhibitors in a dry form by fractional salting out or the proteins by the addition of common salt (NaCl), where the proteaseinhibitors precipitates by the addition of about 150 g salt/litre concentrate with 3 % salt previously added, so that about 70 % saturation is obtained, afterwards
- removing the sediment by centrifugation of filtration and washing with pure salt solution,
- the sediment is dissolved in water, the solution is filtered, and salt removed by dialysis or diafiltration,
- the solution is concentrated under vacuum at approximately 35 °C to a sirup, and the remaining water is removed by freezedrying so that a dry powder is obtained.

## Patentansprüche

1. Verfahren zur Herstellung von Proteasehemmstoffen,
**dadurch gekennzeichnet,**
daß als Ausgangsmaterial für das Herstellungsverfahren Kartoffelsaft aus der Herstellung von Kartoffelstärke wie folgt eingesetzt wird:
- Zugabe von nicht weniger als 200 ppm Schwefeldioxid oder Sulfit, um ein Oxidieren bei der Herstellung von Kartoffelstärke zu verhindern, oder es wird Schwefeldioxid oder Sulfit dem Kartoffelsaft bei der Herstellung von Proteasehemmstoffen zugegeben,
- Einstellen des pH-Werts auf 4,6 oder weniger,
- Erwärmen auf eine Temperatur von 65 - 70° C, d.h. auf die Koagulationstemperatur der inaktiven Proteine (Globuline) im Kartoffelsaft, und zwar mit Hilfe von Wärmetauschern mit einer kontrollierten Oberflächentemperatur von 75 - 80° C, so daß eine örtliche Überhitzung die Proteine der Proteasehemmstoffe nicht beschädigt,
- Abkühlen der Reaktionsmischung nach dem Wärmekoagulieren mit Hilfe eines Kühlungs-Wärmetauschers,
- Verwendung einer Hochgeschwindigkeits-Düsentrennvorrichtung mit einem System zum Absondern des Schlamms zum Entfernen des weichen, schleimigen Koagulats,
- Sammeln und Transportieren der Klarphase aus der Düsentrennvorrichtung zum Konzentrieren,
- Entfernen des Wassers durch Ultrafiltration mit etwa 4 bar Druckabfall an den Membranen und mit hohem Querstrom über die Membranen, bis die vorbestimmte Konzentration erreicht ist, wobei der Aufbau der Konzentration von der Messung des Volumens und von der Bestimmung der Inhibitorenaktivitäten gefolgt wird,
- sowie Zugabe von 7 - 8 % Kochsalz (NaCl) zum Konservieren der Proteine sowie ggfs. anschließende Zugabe von Kochsalz (NaCl) zum Abscheiden aktiver Proteine.

2. Verfahren nach Anspruch 1,
**dadurch gekennzeichnet,**
daß gereinigte Proteasehemmstoffe in trockener Form durch fraktioniertes Aussalzen der Proteine durch Zugabe von Kochsalz (NaCl) hergestellt werden, wobei die Proteasehemmstoffe durch die Zugabe von etwa 150 g Salz pro Liter Konzentrat bei vorgehender Zugabe von 3 % Salz ausscheiden, so daß etwa 70 % Sättigung erreicht werden, worauf
- das Sediment durch Zentrifugieren oder Filtrieren und Waschen mit reiner Salzlösung entfernt wird,
- das Sediment in Wasser gelöst wird, die Lösung filtriet wird und das Salz durch Dialyse oder durch Diafiltrieren entfernt wird, und
- die Lösung unter Vakuum bei etwa 35° C zu Sirup konzentriert wird und das verbleibende Wasser durch Gefriertrocknung entfernt wird, so daß ein Trockenpulver erhalten wird.

## Revendications

1. Procédé de production d'inhibiteurs de la protéase caractérisé en ce qu'on utilise, en tant que produit brut destiné au procédé, du jus de pommes de terre provenant de la production de l'amidon de pommes de terre d'une façon telle que le procédé comprend:
- l'addition d'au moins 200 ppm de dioxyde de soufre ou de sulfite pour empêcher l'oxydation lors de la production de l'amidon de pommes de terre ou l'addition de dioxyde de soufre ou de sulfite au jus de pommes de terre lors de la production des inhibiteurs de la protéase,
- l'ajustement du pH à 4,6 ou moins,
- le chauffage à 65-70°C, ce qui constitue la température de coagulation des protéines inactives (globulines) dans le jus de pommes de terre, avec des échageurs thermiques présentant une température de surface contrôlée de 75-80°C, de sorte qu'un surchauffage local n'endommage pas les protéines inhibitrices de la protéase,
- le refroidissement du mélange réactionnel après la coagulation thermique à l'aide d'un échangeur thermique de refroidissement,
- l'utilisation d'un séparateur à buse à grande vitesse présentant un système pour rejeter l'écume pour se débarrasser du coagulant doux et visqueux,
- le recueil et le transport de la phase limpide à partir du séparateur à buse pour une concentration,
- l'élimination de l'eau par ultrafiltration à une chute de pression d'environ 4 bar de part et d'autre des membranes et à un courant transversal élevé sur les membranes jusqu'à ce qu'on obtienne la concentration prédéterminée, l'établissement de la concentration étant suivi par la mesure des volumes et la détermination des activités des inhibiteurs et
- l'addition de 7 à 8 % de sel de cuisine (NaCl) pour conserver les protéines et en tant que partie de l'addition subséquente de sels nécessaire pour précipiter les protéines actives en cas de besoin.

2. Procédé selon la revendication 1, caractérisé en ce que le mode opératoire comprend également la production d'inhibiteurs de la protéase purifiés sous une forme sèche par un relargage fractionné des proféines par l'addition de sel de cuisine (NaCl) où les inhibiteurs de la protéase précipitent par addition d'environ 150 g de sel/l de concentré avec 8 % de sel ajouté au préalable, de sorte qu'on obtient environ 70% de saturation, puis
- l'élimination du sédiment par centrifugation ou filtration et le lavage avec une solution pure de sel,
- la dissolution du sédiment dans l'eau, la filtration de la solution et l'élimination du sel par dialyse ou diafiltration,
- la concentration de la solution sous vide à approximativement 35°C en un sirop et l'élimination de l'eau restante par lyophilisation, de sorte qu'on obtient une poudre sèche.
